# EUROPEAN PATENT SPECIFICATION

(11) **EP 4 450 125 B1**
(45) Date of publication and mention of the grant of the patent: **18.02.2026**
(21) Application number: 23186537.9
(22) Date of filing: 19.07.2023
(51) Int. Cl.: A61N 7/02, A61B 17/225, A61B 8/00, A61B 18/00

(54) **ULTRASONIC TRANSMISSION MEDIUM CIRCULATION SYSTEM**
ULTRASCHALLÜBERTRAGUNGSMEDIUMZIRKULATIONSSYSTEM
SYSTÈME DE CIRCULATION DE MILIEU DE TRANSMISSION ULTRASONORE

(30) Priority: 17.04.2023 KR 20230050107
(43) Date of publication of application: 23.10.2024
(73) Proprietor: IMGT Co, Ltd., Seongnam-si, Gyeonggi-do 13605 (KR)
(72) Inventor: SON, Keon Ho, 13462 Gyeonggi-do (KR); YU, Young Bok, 02726 Seoul (KR); HAN, Kyu Hoon, 13597 Gyeonggi-do (KR)
(74) Representative: Sander, Rolf

(56) References cited:
- US-A- 4 977 888
- US-A- 5 095 907

## Description

### CROSS-REFERENCE TO RELATED APPLICATION(S)

This application claims the benefit under 35 USC 9119(a) of Korean Patent Application No. 10-2023-0050107, filed on April 17, 2023, in the Korean Intellectual Property Office.

### BACKGROUND

### 1. Field

The following description relates to a treatment technology using ultrasound, and more particularly, to a technology of processing an ultrasonic transmission medium.

### 2. Description of Related Art

Ultrasound signals may be used in the treatment of biological tissues, such as cancer, tumors, lesions, and the like. Treatment with ultrasound is a method of treating a lesion by emitting ultrasound signals to the lesion of the human body. Ultrasound treatment may cause less trauma of a patient, compared to general surgical treatment or chemotherapy, and realize non-invasive treatment. Examples of the application of ultrasound treatment include liver cancer, bone sarcoma, breast cancer, pancreatic cancer, kidney cancer, soft tissue tumors, pelvic tumors, and the like.

US 4 977 888 A (RIETTER JOSEF [DE] ET AL) discloses an apparatus for disintegrating calculi in a body of a life form comprises at least one shockwave generator.

US 5 095 907 A (KUDO NOBUKI [JP] ET AL) discloses ab acoustic wave therapy apparatus.

### SUMMARY

The subject matter of the present invention is defined in independent claim 1. Embodiments are defined in dependent claim 2 to 8.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

The following description relates to an ultrasonic transmission medium circulation system capable of effectively processing an ultrasonic transmission medium for an ultrasound treatment head and a method therefor are proposed.

In one general aspect, there is provided an ultrasonic transmission medium circulation system of an ultrasound treatment head, the ultrasonic transmission medium circulation system comprising: the circulation system comprising: a fluid tank of open form and a fluid block of closed form configured to store an ultrasonic transmission medium; and a single pump; passages comprising: a first passage connected to an outlet of the fluid tank, a second sub-first passage connected to an inlet of the ultrasound treatment head; a second sub-second passage connected to an inlet of the fluid tank; a third sub-first passage connected to an inlet of the fluid block; a third sub-second passage connected to the inlet of the fluid tank; and a fourth passage connected to an outlet of the fluid block; and the plurality of valves comprising: a first valve configured to selectively open and close the first passage; a second valve configured to selectively open and close the second sub-first passage and the second sub-second passage; a third valve configured to selectively open and close the third sub-first passage and the third sub-second passage; and a fourth valve configured to selectively open and close the fourth passage, the circulation system being one in which a passage is changed by selectively opening and closing each of the valves and the ultrasonic transmission medium is supplied, discharged, and circulated by the pump, wherein the circulation system is configured to perform: a supply function by supplying the ultrasonic transmission medium in the fluid tank to the ultrasound treatment head by the pump; a discharge function by discharging the ultrasonic transmission medium in the ultrasound treatment head to the fluid tank by the pump; and a circulation function by allowing the ultrasonic transmission medium in the ultrasound treatment head to return through the fluid block to the ultrasound treatment head by the pump. The circulation system may be configured to operate in at least two distinct operational modes depending on a material of a membrane of the ultrasound treatment head, said modes including: a first operational mode for when the membrane is an elastic material; and a second operation mode for when the membrane is an inelastic material.

The pump may supply the ultrasonic transmission medium in the fluid tank to the ultrasound treatment head through the first passage and the second sub-first passage, and during the supply of the ultrasonic transmission medium, the first valve may be opened, the second valve may open the second sub-first passage and close the second sub-second passage, the third valve may open the third sub-first and close the third sub-second passage, and the fourth valve may be closed.

The pump may discharge the ultrasonic transmission medium in the ultrasound treatment head to the fluid tank through the third sub-first passage, the fourth passage, and the second sub-second passage, and during the discharge of the ultrasonic transmission medium, the first valve may be closed, the second valve may close the second sub-first passage and open the second sub-second passage, the third valve may open the third sub-first passage and close the third sub-second passage, and the fourth valve may be opened.

The pump may allow the ultrasonic transmission medium in the ultrasound treatment head to return through the fluid block to the ultrasound treatment head via the second sub-first passage, the third sub-first passage, and the fourth passage, and during the circulation of the ultrasonic transmission medium, the first valve may be closed, the second valve may open the second sub-first passage and close the second sub-second passage, the third valve may open the third sub-first passage and close the third sub-second passage, and the fourth valve may be opened.

When the membrane is an inelastic material, the pump may allow the ultrasonic transmission medium in the fluid tank to return through the ultrasound treatment head from the outlet of the fluid tank to the inlet of the fluid tank via the first passage, the second sub-first passage, and the third sub-second passage without supplying and discharging the ultrasonic transmission medium to and from the ultrasound treatment head, and during the circulation of the ultrasonic transmission medium, the first valve may be opened, the second valve may open the second sub-first passage and close the second sub-second passage, the third valve may close the third sub-first passage and open the third sub-second passage, and the fourth valve may be closed.

The circulation system may further include a cooler coupled and installed to the fluid tank and the fluid block to simultaneously cool the ultrasonic transmission medium inside both the fluid tank and the fluid block in the process of supplying, discharging, or circulating the ultrasonic transmission medium by means of the pump.

The circulation system may further include a degassing module configured to degas the ultrasonic transmission medium in the process of circulating the ultrasonic transmission medium by means of the pump.

No methods are claimed.

Other features and aspects will be apparent from the following detailed description, the drawings, and the claims.

### BRIEF DESCRIPTION OF THE DRAWINGS

FIG. 1 is a diagram illustrating the configuration of an ultrasonic transmission medium circulation system of an ultrasound treatment head according to an embodiment of the present invention;
FIG. 2 is a diagram illustrating a process of supplying an ultrasonic transmission medium to an ultrasound treatment head having a membrane made of an elastic material according to an embodiment of the present disclosure;
FIG. 3 is a diagram illustrating a process of discharging an ultrasonic transmission medium from an ultrasound treatment head having a membrane made of an elastic material according to an embodiment of the present disclosure;
FIG. 4 is a diagram illustrating a process of circulating an ultrasonic transmission medium for an ultrasound treatment head having a membrane made of an elastic material according to an embodiment of the present disclosure;
FIG. 5 is a diagram illustrating a processing of circulating an ultrasonic transmission medium for an ultrasound treatment head having a membrane made of an inelastic material according to an embodiment of the present disclosure; and
FIG. 6 is a diagram illustrating a method of circulating an ultrasonic transmission medium by an ultrasonic transmission medium circulation system of an ultrasound treatment head according to an embodiment of the present invention.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The advantages and features of the present invention and the manner of achieving the advantages and features will become apparent with reference to embodiments described in detail below together with the accompanying drawings. However, the present invention may be implemented in many different forms and should not be construed as being limited to the embodiments set forth herein, and the embodiments are provided such that this disclosure will be thorough and complete and will fully convey the scope of the present invention to those skilled in the art, and the present invention is defined only by the scope of the appended claims. The same reference numerals refer to the same components throughout this disclosure.

In the following description of the embodiments of the present invention, if a detailed description of related known functions or configurations is determined to unnecessarily obscure the gist of the present invention, the detailed description thereof will be omitted herein. The terms described below are defined in consideration of the functions in the embodiments of the present invention, and these terms may be varied according to the intent or custom of a user or an operator. Therefore, the definitions of the terms used herein should follow contexts disclosed herein.

It will be understood that each block of the flowchart illustrations, and combinations of blocks in the flowchart illustrations, can be implemented by computer program instructions (execution engine). These computer program instructions can be provided to a processor of a general purpose computer, special purpose computer, or other programmable data processing apparatus to produce a machine, such that the instructions, which execute via the processor of the computer or other programmable data processing apparatus, create means for implementing the functions specified in the flowchart block or blocks.

These computer program instructions may also be stored in a computer usable or computer-readable memory that can direct a computer or other programmable data processing apparatus to function in a particular manner, such that the instructions stored in the computer usable or computer-readable memory produce an article of manufacture including instruction means that implement the function specified in each block of block diagrams or in each step of the flowchart illustrations.

The computer program instructions may also be loaded onto a computer or other programmable data processing apparatus to cause a series of operational steps to be performed on the computer or other programmable apparatus to produce a computer implemented process such that the instructions that execute on the computer or other programmable apparatus provide steps for implementing the functions specified in each block of the block diagram or in each step of the flowchart illustrations.

Further, each block or each step of the flowchart illustrations may represent a module, segment, or portion of code, which includes one or more executable instructions for implementing the specified logical function(s). It should also be noted that in some alternative implementations, the functions noted in the blocks may occur out of the order. For example, two blocks shown in succession may in fact be executed substantially concurrently or the blocks may sometimes be executed in the reverse order, depending upon the functionality involved.

Hereinafter, embodiments of the present invention will be described in detail with reference to the accompanying drawings. However, the present invention may be realized in various forms, and the scope of the present invention is not limited to such embodiments. The embodiments of the present invention are provided to aid those skilled in the art in the explanation and the understanding of the present invention.

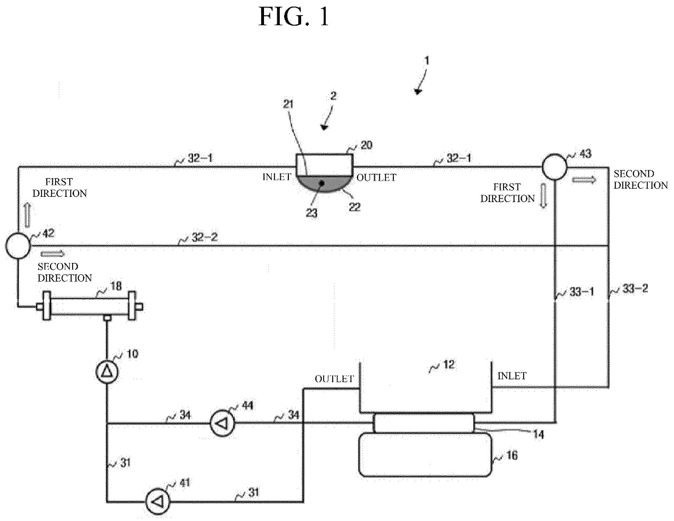

FIG. 1 is a diagram illustrating the configuration of an ultrasonic transmission medium circulation system of an ultrasound treatment head according to an embodiment of the present invention.

Referring to FIG. 1, an ultrasonic transmission medium circulation system 1 is configured to supply, discharge, and circulate an ultrasonic transmission medium for an ultrasound treatment head 1 having an accommodating space 23 in which the ultrasonic transmission medium is accommodated. The ultrasonic transmission medium may be a fluid, such as water.

The ultrasound treatment head 1 includes a treatment transducer 20. The treatment transducer 20 is configured to emit focused ultrasound for treating a patient. The treatment transducer 20 may generate a focused ultrasound signal and focus the focused ultrasound signal to a treatment site. The treatment transducer 20 may have an array structure composed of a plurality of treatment transducers and the plurality of treatment transducers 20 constituting the array may be arranged in a random form.

The treatment transducer 20 may have an ultrasound-emitting surface 21 at a lower end thereof. The ultrasound-emitting surface 21 may be upwardly concave or flat. The ultrasound-emitting surface 21 may be covered by a membrane 22.

The membrane 22 has a structure mounted on a lower end of the ultrasound treatment head 1 to block the ultrasound-emitting surface 21. An accommodation space 23 is formed between the membrane 22 and the ultrasound-emitting surface 21 to accommodate the ultrasonic transmission medium. The ultrasonic transmission medium may consist of degassed water or the like. For example, the membrane 22 may be formed to surround a lower opening and a portion of the lateral surface of the ultrasound treatment head 1, and may be sealed and coupled to the lateral surface of the ultrasound treatment head 1. The membrane 22 may be sealed and coupled to the edge of the ultrasound-emitting surface 21 of the treatment transducer 20, and thus is not limited to the above example.

The membrane 22 has an acoustic impedance similar to that of the ultrasonic transmission medium. The membrane 22 may be an elastic material or an inelastic material. The elastic material may include ethylene propylene diene monomer (EPDM) rubber, latex rubber, silicone rubber, and the like. When the membrane 22 is made of an elastic material, the ultrasound treatment head 1 may adjust a treatment depth. On the contrary, when the membrane 22 is made of an inelastic material, the ultrasound treatment head 1 may have a fixed treatment depth.

An inlet for introducing the ultrasonic transmission medium may be formed on one side of the accommodation space 23 and an outlet for discharging the ultrasonic transmission medium may be formed on the other side of the accommodation space 23.

The ultrasound treatment head 1 is positioned above a patient and emits ultrasound waves through the ultrasound-emitting surface 21 of the treatment transducer 20 in a state in which the membrane 22 is in close contact with the patient's skin. Then, the ultrasound waves are transmitted to a lesion site of the patient through the ultrasonic transmission medium inside the accommodation space 23.

The ultrasonic transmission medium circulation system 1 includes a circulation system including a pump 10, a fluid tank 12, a fluid block 14, a cooler 16, a degassing module 18, a plurality of passages, and a plurality of valves.

The fluid tank 12 and the fluid block 14 accommodate the ultrasonic transmission medium. The fluid tank 12 has an open form, and the fluid block 14 has a closed form.

The pump 10 is configured to supply the ultrasonic transmission medium from the fluid tank 12 to the ultrasound treatment head 2, discharge the ultrasonic transmission medium from the ultrasound treatment head 2 to the fluid tank 12, and circulate the ultrasonic transmission medium in the system. A general system includes a supply pump configured to perform a supply function, a discharge pump configured to perform a discharge function, and a circulation pump configured to perform a circulation function. When a plurality of pumps are used, a system has a complex structure.

However, the ultrasonic transmission medium circulation system 1 according to an embodiment may perform all the supply function, the discharge function, and the circulation function using a single pump 10. To this end, the ultrasonic transmission medium circulation system 1 may be provided with the pump 10, the plurality of passages, and the plurality of valves, as shown in FIG. 1, and may change a flow passage for supply, discharge, or circulation by selectively opening and closing each valve. Accordingly, the supply, discharge, and circulation of the ultrasonic transmission medium may be performed by means of the single pump 10.

The pump 10 may be configured as an electric pump driven by a motor, and may be controlled by a control unit that controls the overall system 1.

The plurality of passages are flow passages for supply, discharge, and circulation of the ultrasonic transmission medium. The plurality of passages may be provided in the form of pipes. The plurality of passages include a first passage 31, a second sub-first passage 32-1, a second sub-second passage 32-2, a third sub-first passage 33-1, a third sub-second passage 33-2, and a fourth passage 34. The first passage 31 is formed with respect to a first valve 41, and connected to an outlet of the fluid tank 12. The second sub-first passage 32-1 and the second sub-second passage 32-2 are formed with respect to a second valve 42, wherein the second sub-first passage 32-1 is connected to an inlet of the ultrasound treatment head 2 and the second sub-second passage 32-2 is connected to an inlet of the fluid tank 12. The third sub-first passage 33-1 and the third sub-second passage 33-2 are formed with respect to a third valve 43, wherein the third sub-first passage 33-1 is connected to an inlet of the fluid block 14 and the third sub-second passage 33-2 is connected to the inlet of the fluid tank 12. The fourth passage 34 is formed with respect to a fourth valve 44, and connected to an outlet of the fluid block 14.

The plurality of valves includes the first valve 41, the second valve 42, the third valve 43, and the fourth valve 44. In this case, the first valve 41 and the fourth valve 44 are controlled in one direction, and the second valve 42 and the third valve 43 are controlled in two directions. The first valve 41 selectively opens and closes the first passage 31. The second valve 42 selectively opens and closes the second sub-first passage 32-1 and the second sub-second passage 32-2. The third valve 43 selectively opens and closes the third sub-first passage 33-1 and the third sub-second passage 33-2. The fourth valve 44 selectively opens and closes the fourth passage 34. The plurality of valves may be controlled by the control unit.

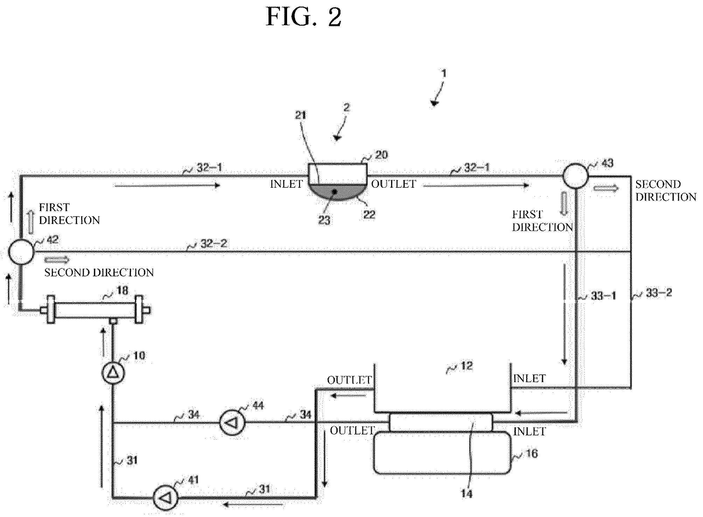

The ultrasonic transmission medium circulation system 1 supplies, discharges, and circulates the ultrasonic transmission medium using the single pump 10. The supply, discharge, and circulation processes of the ultrasonic transmission medium will be described, respectively, with reference to FIGS. 2 to 4.

The ultrasonic transmission medium circulation system 1 according to an embodiment may perform a circulation process by means of the single system 1 regardless of the material (e.g., elastic material or inelastic material) of the membrane 22. That is, instead of distinctively using a separate system suitable for the material of the membrane 22, one system 1 may be used regardless of the material of the membrane 22. To this end, the ultrasonic transmission medium circulation system 1 is driven by changing the passages through selective opening and closing of each valve in the system 1.

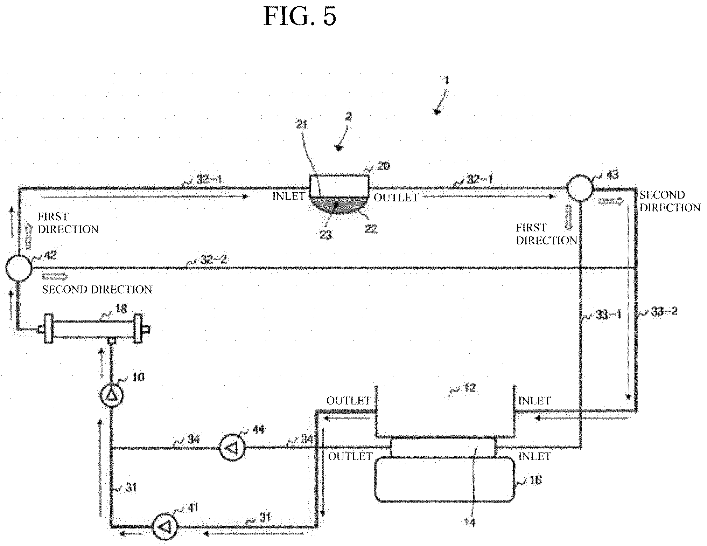

The supply, discharge, and circulation processes of the ultrasonic transmission medium with respect to the ultrasound treatment head having a membrane made of an elastic material will be described below with reference to FIGS. 2 to 4. In addition, the circulation process of the ultrasonic transmission medium with respect to the ultrasound treatment head having a membrane made of an inelastic material will be described with reference to FIG. 5.

The cooler 16 is coupled and installed to the fluid tank 12 and the fluid block 14 to simultaneously cool the ultrasonic transmission medium inside both the fluid tank 12 and the fluid block 14 in the process of supplying, discharging, or circulating the ultrasonic transmission medium by means of the pump 10. As the cooler 16 is integrated with the fluid tank 12 and the fluid block 14, it may be able to simultaneously cool the ultrasonic transmission medium inside both the fluid tank 12 and the fluid block 14. The ultrasonic transmission medium cooled by the cooler 16 during an ultrasound treatment may be supplied into the accommodation space 23 by the pump 10 and mixed with a heated ultrasonic transmission medium inside the accommodation space 23. Therefore, the heated ultrasonic transmission medium inside the accommodation space 23 may be cooled. The cooler 16 may cool the ultrasonic transmission medium inside the fluid tank 12 and the fluid block 14, regardless of the order of the supply, discharge, and circulation processes of the ultrasonic transmission medium, and may automatically or manually operate at a predetermined temperature or higher.

The degassing module 18 degases the ultrasonic transmission medium in the process of circulating the ultrasonic transmission medium by means of the pump 10. The degassing module 18 may be installed between the pump 10 and the second valve 42. Even when bubbles are created in the ultrasonic transmission medium inside the accommodation space 23 during the ultrasound treatment, gas components in the ultrasonic transmission medium may be removed by the degassing module 18 while the ultrasonic transmission medium discharged from the accommodation space 23 is flowing and then supplied back into the accommodation space 23. During the ultrasound treatment, bubbles may be created in the ultrasonic transmission medium by therapeutic ultrasound. The created bubbles may interfere with the transmission of ultrasound waves and change an ultrasound transmission path. In addition, the bubbles may adhere to the ultrasound-emitting surface 21, causing a failure of the treatment transducer 20. Thus, the aforementioned problems may be prevented by degassing the ultrasonic transmission medium.

FIG. 2 is a diagram illustrating a process of supplying an ultrasonic transmission medium to an ultrasound treatment head having a membrane made of an elastic material according to an embodiment of the present disclosure. Here, an arrow represents a flow of an ultrasonic transmission medium.

Referring to FIG. 2, when the membrane 22 is made of an elastic material, the pump 10 supplies the ultrasonic transmission medium in the fluid tank 12 to the ultrasound treatment head 2. For example, the pump 10 supplies the ultrasonic transmission medium to the ultrasound treatment head 2 through the first passage 31 connected to the outlet of the fluid tank 12 and the second sub-first passage 32-1 connected to the inlet of the ultrasound treatment head 2. To this end, the first valve 41 is opened, the second valve 42 opens the second sub-first passage 32-1 and closes the second sub-second passage 32-2, the third valve 43 opens the third sub-first passage 33-1 and closes the third sub-second passage 33-2, and the fourth valve 44 is closed. That is, to supply the ultrasonic transmission medium, the first valve 41 is kept open, the second valve 42 is kept open in the direction of the second sub-first passage 32-1, the third valve 43 is kept open in the direction of the third sub-first passage 33-1, and the fourth valve 44 is kept closed.

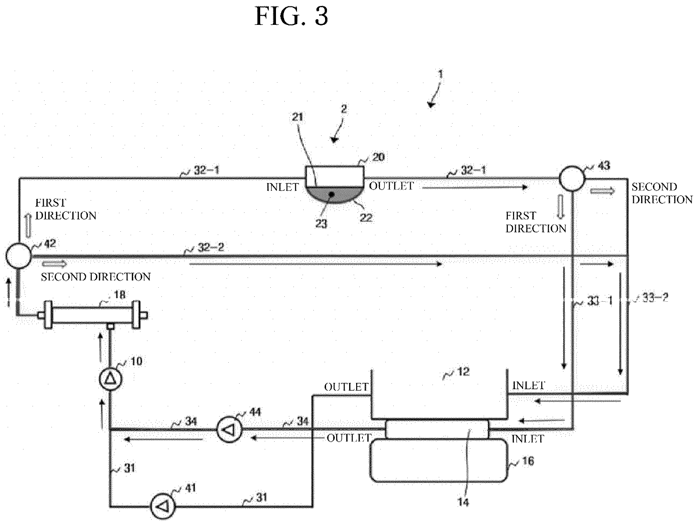

FIG. 3 is a diagram illustrating a process of discharging an ultrasonic transmission medium from an ultrasound treatment head having a membrane made of an elastic material according to an embodiment of the present disclosure. Here, an arrow represents a flow of an ultrasonic transmission medium.

Referring to FIG. 3, when the membrane 22 is made of an elastic material, the pump 10 supplies the ultrasonic transmission medium in the ultrasound treatment head 2 to the fluid tank 12. For example, the pump 10 discharges the ultrasonic transmission medium in the ultrasound treatment head 2 to the fluid tank 12 through the third sub-first passage 33-1 connected to the inlet of the fluid block 14, the fourth passage 34 connected to the outlet of the fluid block 14, and the second sub-second passage 32-2 connected o the inlet of the fluid tank 12. To this end, the first valve 41 is closed, the second valve 42 closes the second sub-first passage 32-1 and opens the second sub-second passage 32-2, the third valve opens the third sub-first passage 33-1 and closes the third sub-second passage 33-2, and the fourth valve 44 is opened. That is, to discharge the ultrasonic transmission medium, the first valve 41 is closed, the second valve 42 is opened in the direction of the second sub-second passage 32-2, the third valve 43 is opened in the direction of the third sub-first passage 33-1, and the fourth valve 44 is opened.

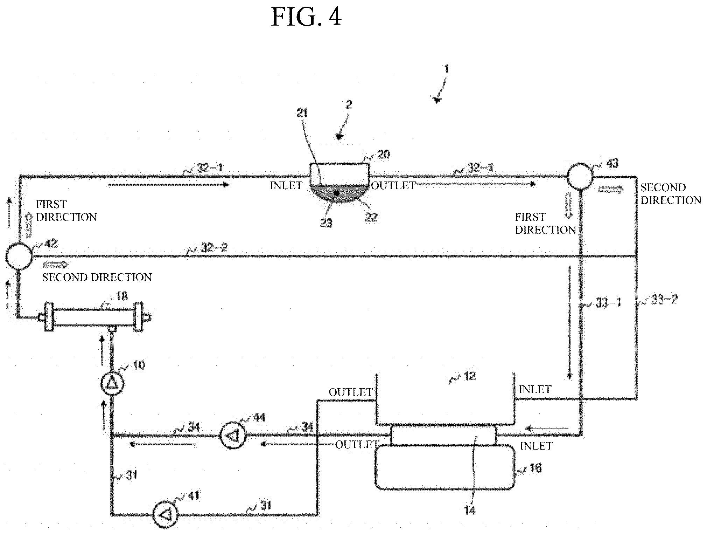

FIG. 4 is a diagram illustrating a process of circulating an ultrasonic transmission medium for an ultrasound treatment head having a membrane made of an elastic material according to an embodiment of the present disclosure. Here, an arrow represents a flow of an ultrasonic transmission medium.

Referring to FIG. 4, when the membrane 22 is made of an elastic material, the pump 10 allows the ultrasonic transmission medium in the ultrasound treatment head 2 to return through the fluid block 14 to the ultrasound treatment head 2. For example, the pump 10 allows the ultrasonic transmission medium in the ultrasound treatment head 2 to return through the fluid block 14 to the ultrasound treatment head 2 via the second sub-first passage 32-1, the third sub-first passage 33-1, and the fourth passage 34. To this end, the first valve 41 is closed, the second valve 42 opens the second sub-first passage 32-1 and closes the second sub-second passage 32-2, the third valve 43 opens the third sub-first passage 33-1 and closes the third sub-second passage 33-2, and the fourth valve 44 is opened. That is, to circulate the ultrasonic transmission medium, the first valve 41 is kept open, the second valve 42 is kept open in the direction of the second sub-first passage 32-1, the third valve 43 is kept open in the direction of the third sub-first passage 33-1, and the fourth valve 44 is kept open.

A degassing process by the degassing module 18 may be performed in the circulation process. The degassing module 18 may continuously operate to remove air from the ultrasonic transmission medium to a level that is considered not to be problematic for use in treatment.

FIG. 5 is a diagram illustrating a processing of circulating an ultrasonic transmission medium for an ultrasound treatment head having a membrane made of an inelastic material according to an embodiment of the present disclosure. Here, an arrow represents a flow of an ultrasonic transmission medium.

Referring to FIG. 5, when the membrane 22 is made of an inelastic material, the pump 10 allows the ultrasonic transmission medium in the fluid tank 12 to return through the ultrasound treatment head 2 to the fluid tank 12. In order to fill the ultrasonic transmission medium in the inelastic membrane 22 made of a hard material, a process of circulating the ultrasonic transmission medium is required without a separate process of supplying and discharging the ultrasonic transmission medium to and from the ultrasound treatment head 2.

For example, the pump 10 circulates the ultrasonic transmission medium in the fluid tank 12 through the ultrasound treatment head 2 from the outlet of the fluid tank 12 back to the inlet of the fluid tank 12 via the first passage 31, the second sub-first passage 32-1, and the third sub-second passage 33-2, without supplying and discharging the ultrasonic transmission medium to and from the ultrasound treatment head 2. During the circulation, the first valve 41 is opened, the second valve 42 opens the second sub-first passage and closes the second sub-second passage 32-2, the third valve 43 closes the third sub-first passage 33-1 and opens the third sub-second passage 33-2, and the fourth valve 44 is closed. That is, to circulate the ultrasonic transmission medium, the first valve 41 is kept open, the second valve 42 is kept open in the direction of the second sub-first passage 32-1, the third valve 43 is kept open in the direction of the third sub-second passage 33-2, and the fourth valve 44 is kept closed.

In the initial circulation process of the above-described circulation process, the ultrasonic transmission medium circulation system 1 transfers air in the ultrasound treatment head 2 into the fluid tank 12. In the subsequent circulation process, the ultrasonic transmission medium circulation system removes the air from the ultrasonic transmission medium using the degassing module 18 up to a level that is considered not to be problematic for use in treatment.

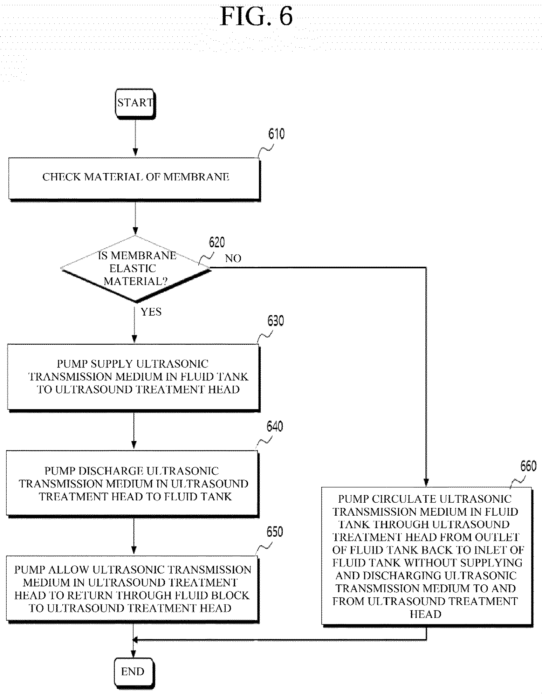

FIG. 6 is a diagram illustrating a method of circulating an ultrasonic transmission medium by an ultrasonic transmission medium circulation system of an ultrasound treatment head according to an embodiment of the present disclosure.

Referring to FIGS. 1 and 6, the ultrasonic transmission medium circulation system 1 checks the material of the membrane 22 in the ultrasound treatment head 2 in 610.

At this time, if it is confirmed in 620 that the membrane 22 is an elastic material, a passage is changed by selectively opening and closing each valve and the ultrasonic transmission medium is supplied, discharged, and circulated by means of the single pump 10.

For example, in the supply process, the pump 10 supplies the ultrasonic transmission medium in the fluid tank 12 to the ultrasound treatment head 2 in 630. For example, the pump 10 may supply the ultrasonic transmission medium to the ultrasound treatment head 2 through the first passage 31 connected to the outlet of the fluid tank 12 and the second sub-first passage 32-1 connected to the inlet of the ultrasound treatment head 2.

In the discharge process, the pump 10 discharges the ultrasonic transmission medium in the ultrasound treatment head 2 to the fluid tank 12 in 640. For example, the pump 10 may discharge the ultrasonic transmission medium in the ultrasound treatment head 2 to the fluid tank 12 through the third sub-first passage 33-1 connected to the inlet of the fluid block 14, the fourth passage 34 connected to the outlet of the fluid block 14, and the second sub-second passage 32-2 connected o the inlet of the fluid tank 12.

In the circulation process, the pump 10 allows the ultrasonic transmission medium in the ultrasound treatment head 2 to return through the fluid block 14 to the ultrasound treatment head 2 in 650. For example, the pump 10 may allow the ultrasonic transmission medium in the ultrasound treatment head 2 to return through the fluid block 14 to the ultrasound treatment head 2 via the second sub-first passage 32-1, the third sub-first passage 33-1, and the fourth passage 34.

On the contrary, if it is confirmed in 620 that the membrane 22 is an inelastic material, the pump 10 circulates the ultrasonic transmission medium in the fluid tank 12 through the ultrasound treatment head 2 back to the fluid tank 12 without supplying and discharging the ultrasonic transmission medium to and from the ultrasound treatment head 2 in 660. For example, the pump 10 circulates the ultrasonic transmission medium in the fluid tank 12 through the ultrasound treatment head 2 from the outlet of the fluid tank 12 back to the inlet of the fluid tank 12 via the first passage 31, the second sub-first passage 32-1, and the third sub-second passage 33-2 connected to the inlet of the fluid tank 12.

The method of circulating an ultrasonic transmission medium described above with reference to FIG. 6 may be performed by a control unit of the system 1.

In the system and the method therefor according to an embodiment, the supply, discharge, and circulation of the ultrasonic transmission medium with respect to the ultrasound treatment head are all possible by means of a single pump, and thus the system may be miniaturized.

In addition, in the system and the method therefor according to an embodiment, the system is driven by changing a passage through selective opening and closing of the valves in the system according to the material of a membrane, so that there is no need to drive a separate system for each membrane material. Therefore, the ultrasonic transmission medium may be processed through a single system regardless of the material of the membrane.

Furthermore, in the system and the method therefor according to an embodiment, the system may be miniaturized by integrating a cooler with a fluid tank.

Heretofore, the present invention has been described by focusing on the exemplary embodiments. It can be understood by those skilled in the art to which the present invention pertains that the present invention can be implemented in modified forms without departing from the essential feature of the present invention. Therefore, the disclosed embodiments should be considered as illustrative rather than determinative. The scope of the present invention is defined by the appended claims.

## Claims

1. An ultrasonic transmission medium circulation system of an ultrasound treatment head, the ultrasonic transmission medium circulation system comprising:
a circulation system (1) comprising:
a fluid tank (12) of open form and a fluid block (14) of closed form configured to store an ultrasonic transmission medium; and
a single pump (10);
passages comprising:
- a first passage (31) connected to an outlet of the fluid tank (12),
- a second sub-first passage (32-1) connected to an inlet of the ultrasound treatment head (2);
- a second sub-second passage (32-2) connected to an inlet of the fluid tank (12);
- a third sub-first passage (33-1) connected to an inlet of the fluid block (14);
- a third sub-second passage (33-2) connected to the inlet of the fluid tank (12); and
- a fourth passage (34) connected to an outlet of the fluid block (14); and
a plurality of valves comprising:
- a first valve (41) configured to selectively open and close the first passage (31);
- a second valve (42) configured to selectively open and close the second sub-first passage (32-1) and the second sub-second passage (32-2);
- a third valve (43) configured to selectively open and close the third sub-first passage (33-1) and the third sub-second passage (33-2); and
- a fourth valve (44) configured to selectively open and close the fourth passage (34), the circulation system being one in which a passage is changed by selectively opening and closing each of the valves and the ultrasonic transmission medium is supplied, discharged,
and circulated by the pump (10),
wherein the circulation system (1) is configured to perform:
a supply function by supplying the ultrasonic transmission medium in the fluid tank (12) to the ultrasound treatment head (2) by the pump (10);
a discharge function by discharging the ultrasonic transmission medium in the ultrasound treatment head (2) to the fluid tank (12) by the pump (10); and
a circulation function by allowing the ultrasonic transmission medium in the ultrasound treatment head (2) to return through the fluid block (14) to the ultrasound treatment head (2) by the pump (10).

2. The ultrasonic transmission medium circulation system of claim 1, wherein the circulation system (1) is configured to operate in at least two distinct operational modes depending on a material of a membrane (22) of the ultrasound treatment head (2), said modes including:
a first operational mode for when the membrane (22) is an elastic material; and
a second operational mode for when the membrane (22) is an inelastic material.

3. The ultrasonic transmission medium circulation system of claim 2, wherein, in the first operational mode for when the membrane (22) is an elastic material, the pump (10) is configured to supply the ultrasonic transmission medium in the fluid tank to the ultrasound treatment head (2) through the first passage (31) and the second sub-first passage (32-1), and
during the supply of the ultrasonic transmission medium, the first valve (41) is configured to be opened, the second valve (42) is configured to open the second sub-first passage (32-1) and close the second sub-second passage (32-2), the third valve (43) is configured to open the third sub-first passage (33-1) and close the third sub-second passage (33-2), and the fourth valve (44) is configured to be closed.

4. The ultrasonic transmission medium circulation system of claim 2, wherein, in the first operational mode for when the membrane (22) is an elastic material, the pump (10) is configured to discharge the ultrasonic transmission medium in the ultrasound treatment head to the fluid tank through the third sub-first passage (33-1), the fourth passage (34), and the second sub-second passage (32-2), and
during the discharge of the ultrasonic transmission medium, the first valve (41) is configured to be closed, the second valve (42) is configured to close the second sub-first passage (32-1) and open the second sub-second passage (32-2), the third valve (43) is configured to open the third sub-first passage (33-1) and close the third sub-second passage (33-2), and the fourth valve (44) is configured to be opened.

5. The ultrasonic transmission medium circulation system of claim 2, wherein, in the first operational mode for when the membrane (22) is an elastic material, the pump (10) is configured to allow the ultrasonic transmission medium in the ultrasound treatment head (2) to return through the fluid block (14) to the ultrasound treatment head via the second sub-first passage (32-1), the third sub-first passage (33-1), and the fourth passage (34), and
during the circulation of the ultrasonic transmission medium, the first valve (41) is configured to be closed, the second valve (42) is configured to open the second sub-first passage (32-1) and close the second sub-second passage (32-2), the third valve (43) is configured to open the third sub-first passage (33-1) and close the third sub-second passage (33-2), and the fourth valve (44) is configured to be opened.

6. The ultrasonic transmission medium circulation system of claim 2, wherein, in the second operational mode for when the membrane (22) of the ultrasound treatment head (2) is an inelastic material, the pump (10) is configured to allow the ultrasonic transmission medium in the fluid tank (12) to return through the ultrasound treatment head (2) from the outlet of the fluid tank to the inlet of the fluid tank via the first passage (31), the second sub-first passage (32-1), and the third sub-second passage (33-2) without supplying and discharging the ultrasonic transmission medium to and from the ultrasound treatment head, and
during the circulation of the ultrasonic transmission medium, the first valve (41) is configured to be opened, the second valve (42) is configured to open the second sub-first passage (32-1) and close the second sub-second passage (32-2), the third valve (43) is configured to close the third sub-first passage (33-1) and open the third sub-second passage (33-2), and the fourth valve (44) is configured to be closed.

7. The ultrasonic transmission medium circulation system of claim 6, wherein the circulation system further comprises a cooler (16) coupled and installed to the fluid tank (12) and the fluid block (14) to simultaneously cool the ultrasonic transmission medium inside both the fluid tank and the fluid block in the process of supplying, discharging, or circulating the ultrasonic transmission medium by means of the pump (10).

8. The ultrasonic transmission medium circulation system of claim 1, wherein the circulation system further comprises a degassing module (18) configured to degas the ultrasonic transmission medium in the process of circulating the ultrasonic transmission medium by means of the pump (10).

## Patentansprüche

1. Ultraschallübertragungsmediumzirkulationssystem eines Ultraschallbehandlungskopfs, das Ultraschallübertragungsmediumzirkulationssystem umfassend:
ein Zirkulationssystem (1), umfassend:
einen Fluidtank (12) von offener Form und einen Fluidblock (14) von geschlossener Form, der dazu konfiguriert ist, ein Ultraschallübertragungsmedium zu speichern; und
eine einzelne Pumpe (10);
Kanäle, umfassend:
- einem ersten Kanal (31), der mit einem Auslass des Fluidtanks (12) verbunden ist,
- einen zweiten, dem ersten untergeordneten Kanal (32-1), der mit einem Einlass des Ultraschallbehandlungskopfs (2) verbunden ist;
- einen zweiten, dem zweiten untergeordneten Kanal (32-2), der mit einem Einlass des Fluidtanks (12) verbunden ist;
- einen dritten, dem ersten untergeordneten Kanal (33-1), der mit einem Einlass des Fluidblocks (14) verbunden ist;
- einen dritten, dem zweiten untergeordneten Kanal (33-2), der mit dem Einlass des Fluidtanks (12) verbunden ist; und
- einen vierten Kanal (34), der mit einem Auslass des Fluidblocks (14) verbunden ist; und
eine Vielzahl von Ventilen, umfassend:
- ein erstes Ventil (41), das konfiguriert ist, um den ersten Kanal (31) selektiv zu öffnen und zu schließen;
- ein zweites Ventil (42), das konfiguriert ist, um den zweiten, dem ersten untergeordneten Kanal (32-1) und den zweiten, dem zweiten untergeordneten Kanal (32-2) selektiv zu öffnen und zu schließen;
- ein drittes Ventil (43), das konfiguriert ist, um den dritten, dem ersten untergeordneten Kanal (33-1) und den dritten, dem zweiten untergeordneten Kanal (33-2) selektiv zu öffnen und zu schließen; und
- ein viertes Ventil (44), das konfiguriert ist, um den vierten Kanal (34) selektiv zu öffnen und zu schließen, wobei das Zirkulationssystem eines ist, in welchem ein Kanal durch selektives Öffnen und Schließen jedes der Ventile geändert wird und das Ultraschallübertragungsmedium mittels der Pumpe (10) zugeführt, abgeführt und zirkuliert gelassen wird,
wobei das Zirkulationssystem (1) konfiguriert ist, um Folgendes durchzuführen:
eine Zufuhrfunktion durch Zuführen des Ultraschallübertragungsmediums in dem Fluidtank (12) zu dem Ultraschallbehandlungskopf (2) mittels der Pumpe (10);
eine Abfuhrfunktion durch Abführen des Ultraschallübertragungsmediums in dem Ultraschallbehandlungskopf (2) an den Fluidtank (12) mittels der Pumpe (10); und
eine Zirkulationsfunktion, indem dem Ultraschallübertragungsmedium im Ultraschallbehandlungskopf (2) ermöglicht wird, durch den Fluidblock (14) zum Ultraschallbehandlungskopf (2) mittels der Pumpe (10) zurückzukehren.

2. Ultraschallübertragungsmediumzirkulationssystem nach Anspruch 1, wobei das Zirkulationssystem (1) konfiguriert ist, um in zumindest zwei verschiedenen Betriebsmodi abhängig von einem Material einer Membran (22) des Ultraschallbehandlungskopfs (2) zu arbeiten, wobei die Modi Folgendes einschließen:
einen ersten Betriebsmodus, wenn die Membran (22) ein elastisches Material ist; und
einen zweiten Betriebsmodus, wenn die Membran (22) ein unelastisches Material ist.

3. Ultraschallübertragungsmediumzirkulationssystem nach Anspruch 2, wobei in dem ersten Betriebsmodus, wenn die Membran (22) ein elastisches Material ist, die Pumpe (10) konfiguriert ist, um das Ultraschallübertragungsmedium in dem Fluidtank dem Ultraschallbehandlungskopf (2) durch den ersten Kanal (31) und den zweiten, dem ersten untergeordneten Kanal (32-1) zuzuführen, und
während der Zufuhr des Ultraschallübertragungsmediums das erste Ventil (41) konfiguriert ist, um geöffnet zu werden, das zweite Ventil (42) konfiguriert ist, um den zweiten, dem ersten untergeordneten Kanal (32-1) zu öffnen und den zweiten, dem zweiten untergeordneten Kanal (32-2) zu schließen, das dritte Ventil (43) konfiguriert ist, um den dritten, dem ersten untergeordneten Kanal (33-1) zu öffnen und den dritten, dem zweiten untergeordneten Kanal (33-2) zu schließen, und das vierte Ventil (44) konfiguriert ist, um geschlossen zu sein.

4. Ultraschallübertragungsmediumzirkulationssystem nach Anspruch 2, wobei in dem ersten Betriebsmodus, wenn die Membran (22) ein elastisches Material ist, die Pumpe (10) konfiguriert ist, um das Ultraschallübertragungsmedium in dem Ultraschallbehandlungskopf an den Fluidtank durch den dritten, dem ersten untergeordneten Kanal (33-1), den vierten Kanal (34) und den zweiten, dem zweiten untergeordneten Kanal (32-2) abzugeben, und
während des Abführens des Ultraschallübertragungsmediums das erste Ventil (41) konfiguriert ist, um geschlossen zu sein, das zweite Ventil (42) konfiguriert ist, um den zweiten, dem ersten untergeordneten Kanal (32-1) zu schließen und den zweiten, dem zweiten untergeordneten Kanal (32-2) zu öffnen, das dritte Ventil (43) konfiguriert ist, um den dritten, dem ersten untergeordneten Kanal (33-1) zu öffnen und den dritten, dem zweiten untergeordneten Kanal (33-2) zu schließen, und das vierte Ventil (44) konfiguriert ist, um geöffnet zu werden.

5. Ultraschallübertragungsmediumzirkulationssystem nach Anspruch 2, wobei in dem ersten Betriebsmodus, wenn die Membran (22) ein elastisches Material ist, die Pumpe (10) konfiguriert ist, um dem Ultraschallübertragungsmedium in dem Ultraschallbehandlungskopf (2) zu ermöglichen, durch den Fluidblock (14) zu dem Ultraschallbehandlungskopf über den zweiten, dem ersten untergeordneten Kanal (32-1), dem dritten, dem ersten untergeordneten Kanal (33-1) und dem vierten Kanal (34) zurückzukehren, und
während der Zirkulation des Ultraschallübertragungsmediums das erste Ventil (41) konfiguriert ist, um geschlossen zu werden, das zweite Ventil (42) konfiguriert ist, um den zweiten, dem ersten untergeordneten Kanal (32-1) zu öffnen und den zweiten, dem zweiten untergeordneten Kanal (32-2) zu schließen, das dritte Ventil (43) konfiguriert ist, um den dritten, dem ersten untergeordneten Kanal (33-1) zu öffnen, und den dritten, dem zweiten untergeordneten Kanal (33-2) zu schließen, und das vierte Ventil (44) konfiguriert ist, um geöffnet zu werden.

6. Ultraschallübertragungsmediumzirkulationssystem nach Anspruch 2, wobei in dem zweiten Betriebsmodus, wenn die Membran (22) des Ultraschallbehandlungskopfes (2) ein unelastisches Material ist, die Pumpe (10) konfiguriert ist, um dem Ultraschallübertragungsmedium in dem Fluidtank (12) zu ermöglichen, durch den Ultraschallbehandlungskopf (2) von dem Auslass des Fluidtanks zu dem Einlass des Fluidtanks über den ersten Kanal (31), den zweiten, dem ersten untergeordneten Kanal (32-1) und den dritten, dem zweiten untergeordneten Kanal (33-2) zurückzukehren, ohne das Ultraschallübertragungsmedium dem Ultraschallbehandlungskopf zuzuführen und von diesem abzuführen, und
während der Zirkulation des Ultraschallübertragungsmediums das erste Ventil (41) konfiguriert ist, um geöffnet zu werden, das zweite Ventil (42) konfiguriert ist, um den zweiten, dem ersten untergeordneten Kanal (32-1) zu öffnen und den zweiten, dem zweiten untergeordneten Kanal (32-2) zu schließen, das dritte Ventil (43) konfiguriert ist, um den dritten, dem ersten untergeordneten Kanal (33-1) zu schließen und den dritten, dem zweiten untergeordneten Kanal (33-2) zu öffnen, und das vierte Ventil (44) konfiguriert ist, um geschlossen zu werden.

7. Ultraschallübertragungsmediumzirkulationssystem nach Anspruch 6, wobei das Zirkulationssystem ferner einen Kühler (16) umfasst, der an den Fluidtank (12) und den Fluidblock (14) gekoppelt und installiert ist, um gleichzeitig das Ultraschallübertragungsmedium sowohl innerhalb des Fluidtanks als auch des Fluidblocks in dem Prozess des Zuführens, Abführens oder Zirkulierens des Ultraschallübertragungsmediums mithilfe der Pumpe (10) zu kühlen.

8. Ultraschallübertragungsmediumzirkulationssystem nach Anspruch 1, wobei das Zirkulationssystem ferner ein Entgasungsmodul (18) umfasst, das konfiguriert ist, um das Ultraschallübertragungsmedium in dem Vorgang des Zirkulierens des Ultraschallübertragungsmediums mithilfe der Pumpe (10) zu entgasen.

## Revendications

1. Système de circulation de milieu de transmission ultrasonore d'une tête de traitement aux ultrasons, le système de circulation de milieu de transmission ultrasonore comprenant :
un système de circulation (1) comprenant :
un réservoir de fluide (12) de forme ouverte et un bloc de fluide (14) de forme fermée conçu pour stocker un milieu de transmission ultrasonore ; et
une seule pompe (10) ;
des passages comprenant :
- un premier passage (31) raccordé à une sortie du réservoir de fluide (12),
- un deuxième sous-premier passage (32-1) raccordé à une entrée de la tête de traitement aux ultrasons (2) ;
- un deuxième sous-second passage (32-2) raccordé à une entrée du réservoir de fluide (12) ;
- un troisième sous-premier passage (33-1) raccordé à une entrée du bloc de fluide (14) ;
- un troisième sous-second passage (33-2) raccordé à l'entrée du réservoir de fluide (12) ; et
- un quatrième passage (34) raccordé à une sortie du bloc de fluide (14) ; et
une pluralité de vannes comprenant :
- une première vanne (41) conçue pour ouvrir et fermer de manière sélective le premier passage (31) ;
- une deuxième vanne (42) conçue pour ouvrir et fermer de manière sélective le deuxième sous-premier passage (32-1) et le deuxième sous-second passage (32-2) ;
- une troisième vanne (43) conçue pour ouvrir et fermer de manière sélective le troisième sous-premier passage (33-1) et le troisième sous-second passage (33-2) ; et
- une quatrième vanne (44) conçue pour ouvrir et fermer de manière sélective le quatrième passage (34), le système de circulation étant un système dans lequel un passage est changé en ouvrant et en fermant de manière sélective chacune des vannes et le milieu de transmission ultrasonore étant alimenté, déchargé et mis en circulation par la pompe (10),
ledit système de circulation (1) étant conçu pour réaliser :
une fonction d'alimentation en délivrant le milieu de transmission ultrasonore dans le réservoir de fluide (12) à la tête de traitement aux ultrasons (2) par la pompe (10) ;
une fonction de décharge en déchargeant le milieu de transmission ultrasonore dans la tête de traitement aux ultrasons (2) vers le réservoir de fluide (12) par la pompe (10) ; et
une fonction de circulation en permettant au milieu de transmission ultrasonore dans la tête de traitement aux ultrasons (2) de revenir par l'intermédiaire du bloc de fluide (14) à la tête de traitement aux ultrasons (2) par la pompe (10).

2. Système de circulation de milieu de transmission ultrasonore selon la revendication 1, ledit système de circulation (1) étant conçu pour fonctionner dans au moins deux modes de fonctionnement distincts en fonction d'un matériau d'une membrane (22) de la tête de traitement aux ultrasons (2), lesdits modes comprenant :
un premier mode de fonctionnement dans le cas où la membrane (22) est un matériau élastique ; et
un second mode de fonctionnement dans le cas où la membrane (22) est un matériau non élastique.

3. Système de circulation de milieu de transmission ultrasonore selon la revendication 2, dans le premier mode de fonctionnement dans le cas où la membrane (22) est un matériau élastique, ladite pompe (10) étant conçue pour alimenter le milieu de transmission ultrasonore dans le réservoir de fluide à la tête de traitement aux ultrasons (2) à travers le premier passage (31) et le deuxième sous-premier passage (32-1), et
pendant l'alimentation du milieu de transmission ultrasonore, ladite première vanne (41) étant conçue pour être ouverte, ladite deuxième vanne (42) étant conçue pour ouvrir le deuxième sous-premier passage (32-1) et fermer le deuxième sous-second passage (32-2), ladite troisième vanne (43) étant conçue pour ouvrir le troisième sous-premier passage (33-1) et fermer le troisième sous-second passage (33-2), et ladite quatrième vanne (44) étant conçue pour être fermée.

4. Système de circulation de milieu de transmission ultrasonore selon la revendication 2, dans le premier mode de fonctionnement dans le cas où la membrane (22) est un matériau élastique, ladite pompe (10) étant conçue pour décharger le milieu de transmission ultrasonore dans la tête de traitement aux ultrasons vers le réservoir de fluide à travers le troisième sous-premier passage (33-1), le quatrième passage (34) et le deuxième sous-second passage (32-2), et
pendant la décharge du milieu de transmission ultrasonore, ladite première vanne (41) étant conçue pour être fermée, ladite deuxième vanne (42) étant conçue pour fermer le deuxième sous-premier passage (32-1) et ouvrir le deuxième sous-second passage (32-2), ladite troisième vanne (43) étant conçue pour ouvrir le troisième sous-premier passage (33-1) et fermer le troisième sous-second passage (33-2), et ladite quatrième vanne (44) étant conçue pour être ouverte.

5. Système de circulation de milieu de transmission ultrasonore selon la revendication 2, dans le premier mode de fonctionnement dans le cas où la membrane (22) est un matériau élastique, ladite pompe (10) étant conçue pour permettre au milieu de transmission ultrasonore dans la tête de traitement aux ultrasons (2) de revenir à travers le bloc de fluide (14) à la tête de traitement aux ultrasons par le deuxième sous-premier passage (32-1), le troisième sous-premier passage (33-1) et le quatrième passage (34), et
pendant la circulation du milieu de transmission ultrasonore, ladite première vanne (41) étant conçue pour être fermée, ladite deuxième vanne (42) étant conçue pour ouvrir le deuxième sous-premier passage (32-1) et fermer le deuxième sous-second passage (32-2), ladite troisième vanne (43) étant conçue pour ouvrir le troisième sous-premier passage (33-1) et fermer le troisième sous-second passage (33-2), et ladite quatrième vanne (44) étant conçue pour être ouverte.

6. Système de circulation de milieu de transmission ultrasonore selon la revendication 2, dans le second mode de fonctionnement dans le cas où la membrane (22) de la tête de traitement aux ultrasons (2) est un matériau non élastique, ladite pompe (10) étant conçue pour permettre au milieu de transmission ultrasonore dans le réservoir de fluide (12) de revenir à travers la tête de traitement aux ultrasons (2) de la sortie du réservoir de fluide à l'entrée du réservoir de fluide par le premier passage (31), le deuxième sous-premier passage (32-1) et le troisième sous-second passage (33-2) sans alimenter et décharger le milieu de transmission ultrasonore vers et depuis la tête de traitement aux ultrasons, et
pendant la circulation du milieu de transmission ultrasonore, ladite première vanne (41) étant conçue pour être ouverte, ladite deuxième vanne (42) étant conçue pour ouvrir le deuxième sous-premier passage (32-1) et fermer le deuxième sous-second passage (32-2), ladite troisième vanne (43) étant conçue pour fermer le troisième sous-premier passage (33-1) et ouvrir le troisième sous-second passage (33-2), et ladite quatrième vanne (44) étant conçue pour être fermée.

7. Système de circulation de milieu de transmission ultrasonore selon la revendication 6, ledit système de circulation comprenant en outre un refroidisseur (16) couplé au réservoir de fluide (12) et au bloc de fluide (14) et installé sur ceux-ci pour refroidir simultanément le milieu de transmission ultrasonore à l'intérieur à la fois du réservoir de fluide et du bloc de fluide dans le processus d'alimentation, de décharge ou de circulation du milieu de transmission ultrasonore au moyen de la pompe (10).

8. Système de circulation de milieu de transmission ultrasonore selon la revendication 1, ledit système de circulation comprenant en outre un module de dégazage (18) conçu pour dégazer le milieu de transmission ultrasonore dans le processus de circulation du milieu de transmission ultrasonore au moyen de la pompe (10).
